# EUROPEAN PATENT SPECIFICATION

(11) **EP 1 515 729 B1**
(45) Date of publication and mention of the grant of the patent: **20.02.2008**
(21) Application number: 03740232.8
(22) Date of filing: 12.06.2003
(51) Int. Cl.: A61K 31/724, C08B 37/16, A61P 31/00

(54) **QUATERNISED AMMONIUM CYCLODEXTRIN COMPOUNDS**
QUATERNISIERTE AMMONIUMCYCLODEXTRINVERBINDUNGEN
COMPOSES DE CYCLODEXTRINE D'AMMONIUM QUARTERNAIRE

(30) Priority: 13.06.2002 EP 02013074; 20.12.2002 EP 02028554
(43) Date of publication of application: 23.03.2005
(73) Proprietor: Novartis AG, 4056 Basel (CH); Novartis Pharma GmbH, 1230 Wien (AT)
(72) Inventor: KIS, Georg, Ludwig, CH-8273 Triboltingen (CH); SCHOCH, Christian, CH-4132 Muttenz (CH); SZEJTLI, Jozsef, H-1028 Budapest (HU)
(74) Representative: Morris, Clive Sydney
(86) International application number: PCT/EP2003/006192
(87) International publication number: WO 2003/105867

(56) References cited:
- EP-A- 0 387 681
- WO-A-96/11003
- WO-A-97/10805
- US-A- 3 453 257
- US-A- 5 241 059
- LOFTSSON T ET AL: "CYCLODEXTRIN SOLUBILIZATION OF ETH-615, A ZWITTERIONIC DRUG" DRUG DEVELOPMENT AND INDUSTRIAL PHARMACY, NEW YORK, NY, US, vol. 24, no. 4, 1998, pages 365-370, XP008023012 ISSN: 0363-9045 cited in the application

## Description

The present invention relates to novel uses of quaternized ammonium cyclodextrin compounds (hereinafter also QACD compounds) having the formula (I): 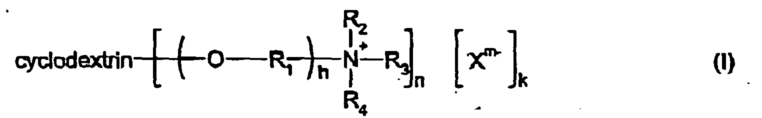 and to novel preservatives particularly a drug dosage system comprising said compounds and their uses.

The compounds of formula (I), wherein 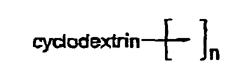 cyclodextrin⁅ ]ₙ represents a n-valent residue derived from a cyclodextrin (=CD) compound by removing n of its hydroxyl groups;
n is a number greater than 0 and represents the average number of substituents of formula 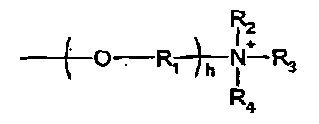 per molecule of said compound;
h is 0 or 1;
R₁ is a di-valent group selected from alkylene, hydroxy alkylene, halogeno alkylene, monocyclic aralkylene, cycloalkylene and phenylene;
R_{2,} R₃ and R₄ are each independently of one another a group selected from alkyl, cycloalkyl, aryl, aralkyl, and cycloheteryl;
X^{m-} is a m-fold negatively charged anion;
m is an integer being equal or greater than 1; and
k is n/m,
are known and described, for instance, in U.S.-Patent No. 3,453,257 (index h = 1) or U.S.-Patent 5,241,059 (index h = 0).

The compounds of formula (I) are desaibed in said references as being better soluble in water than non-cationic cyclodextrin compounds like, for example, beta-cyclodextrin itself.

Based on these findings, US-Patent 5,241,059 states in very general terms that compounds of formula (I) may be useful for making pharmaceuticals, for example. In a more recent investigation (T. Loftsson et al., Drug Development and Industrial Pharmacy, 24(4), 365-370 (1998)) it has furthermore been found that cationic cyclodextrin derivatives like e.g. 2-hydroxy-3-trimethyl-ammoniopropyl-beta-cydodextrin, have a reduced solubilizing effect on organic compounds in water in comparison to uncharged cyclodextrins, like 2-hydroxypropyl-beta-cydodextrin or randomly methylated beta-cyclodextrin, in particular on zwitterionic compounds, especially on the anti-inflammatory drug ETH-615 having the following formula:

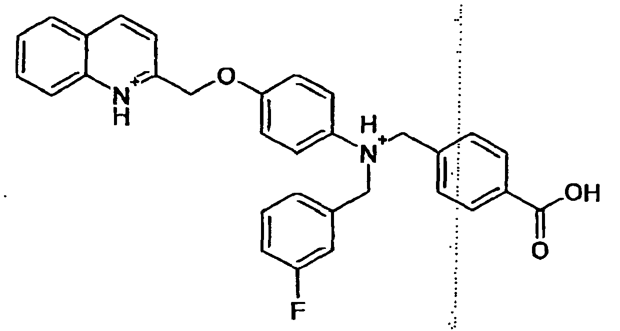

EP 387 681 describes pharmaceutical compositions comprising ammonium salts of polycyclodextrins and their use as hypocholesteremic agents.

In a first aspect, the present invention is based on the surprising findings that the compounds of formula (I) are potent anti-microbial agents, in particular effective against bacteria and fungi. Furthermore, said compounds are also useful against viruses. The compounds of formula (I) exhibit a very low toxicity as shown, for example, by a LD₅₀ in mice of 750 - 1500 mg/kg) and exhibit excellent tolerability. Based on their anti-microbial activity the compounds of formula (I) can be used, on one side, as anti-infective drugs for the treatment of infective diseases caused by the presence of bacteria, fungi or viruses and, on the other side, as potent preservatives for all types of compositions which require preservation, in particular pharmaceutical compositions.

Therefore, in a first aspect the instant invention relates to the use of compounds of the aforementioned formula (I) in the preparation of an anti-infective medicament, that means a medicament for the treatment of bacterial, fungal and viral infections, in particular bacterial and fungal infections of a mammal, particularly a human, in need of such treatment.

The compounds of formula (I) represent a new class of anti-infective agents, and may therefore be particular valuable in view of controlling infections of microorganisms, which are resistant against one or more of anti-infective agents presently in use, like antibiotics. Furthermore said compounds may provide a further alternative for treating patients who cannot tolerate one or more known anti-infective compounds or are allergic: against the known compounds.

The compounds of formula (I) are substances derived from the welt-known cyclodextrins, a group of homologous oligosaccharides. As also well known, cyclodextrins are homologous cyclic molecules containing 6 or more, especially 6, 7 or 8 alfa-D-glucopyranose units linked together at the 1,4 positions as in amylose. When the number of alfa-D-glucopyranose units is 6, the molecule is known as an alfa-cyclodextrin, when the number of alfa-D-glucopyranose units is 7, the molecule is known as a beta-cyclodextrin; and whein this number is 8, the molecule is known as gamma-cyclodextrin. For the purposes of this application the term "cyclodextrin" is intended to include the aforementioned forms, as well as other corresponding cyclic molecules that have a still larger number of alfa-D-glucopyranose units in the molecule, and, as well, mixtures of these and, optionally, other homologs. The various homologous cyclodextrins, having from six to eight units, or more, and their mixtures, may be used as equivalent materials for the purposes of this invention. In practice, there may be little reason for separating the various fractions, and the cyclodextrin employed may contain a preponderance of specific cyclodextrin, e.g. beta-cyclodextrin. No distinction is intended between the various homologous cyclodextrins or their mixtures unless otherwise indicated, when using the term "cyclodextrin".

The term quaternized ammonium cyclodextrin compound of the instant invention also includes the corresponding derivatives wherein one or more of the free hydroxyl groups have been etherified, in particular corresponding methyl ether derivatives.

Preferred cyclodextrin compounds according to the invention include the compounds of formula (Ia): 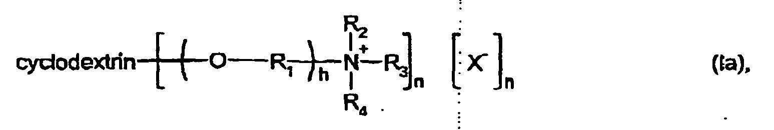 wherein X⁻ is a simple-charged anion and the other residues and h and n have the meaning as in formula (I).

In formula (I) and (Ia) R₁ may be an alkylene, a hydroxy alkylene, an otherwise substituted alkylene, an aralkylene, a cycloalkylene, or a phenylene radical. Thus, R₁ may be, for instance, a branched or straight chain C₁-C₈alkylene, for instance methylene, ethylene, a propylene, butylene, pentylene, hexylene or an octylene group etc. The foregoing and other alkylene radicals also may be substituted in one or more places, for instance, by a hydroxyl, alkoxy, aryl or cycloalkylene radical derived from, for example, cyclopropane, cyclobutane and higher homologues. R₁ may also represent a phenylene radical which may be substituted, if desired e.g. by alkoxy, alkyl, preferably C₁-C₄alkyl or halogen.

R₂, R₃ and R₄ may be different or the same, and may be alkyl, aryl, aralkyl, cycloalkyl, cycloheteryl.

Thus, R₂, R₃ and R₄ may, for instance, be C₁-C₁₈alkyl, like methyl, ethyl, n-propyl, i-propyl, n-butyl, sec-butyl, t-butyl, a branched or straight chain pentyl, hexyl, heptyl, octyl, decyl or octadecyl and the like alkyl radicals, and may also be substituted by one or more substituents, preferably one or more hydroxyl or halogen substituent. Preferably alkyl means C₁-C₁₀alkyl, for instance C₁-C₈alkyl, especially C₁-C₄alkyl, very specially methyl.

In the meaning cycloalkyl R₂, R₃ and R₄ may, preferably, represent C₃-C₆cycloalkyl, like for example, cyclopropyl, cyclobutyl, cyclopentyl or cyclohexyl, which may also be substituted by one or more substiuents, e.g. by C₁-C₄alkyl, hydroxyl or halogen.

As aryl group R₂, R₃ and R₄, are preferably phenyl which may be substituted, for instance by alkyl, in particular C₁-C₄alkyl, or halogen.

In the meaning cycloheteryl R₂, R₃ and R₄ represent the residue of a heterocyclic group, for instance morpholinyl, pyridyl, pyrrolidyl, furfuryl, imidazolidyl, imidazolyl and the like.

X^{m-} is a m-fold negatively charged anion, preferably a halide, including bromide, chloride and iodide, nitrate, phosphate, sulfate, formate, acetate, butyrate, oleate, stearate, benzoate anion, or the like, whereas [X⁻] is a simple-charged anion, preferably also one selected from those above.

In preferred compounds of formula (I) or (Ia) R₂, R₃ and R₄ represent same or different C₁-C₁₈alkyl radicals. Specific preference is thereby given to those alkyl radicals already mentioned above.

As is known, each cyclodextrin molecule of a compound of formula (I) or (Ia) may have a different degree of substitution. The total number of quaternized ammonium groups in the molecule corresponding to the index n, may range from 1 to the maximum number of hydroxyl groups of the base cyclodextrin, that means theoretically up to 18 in case of alfa-cyclodextrin, 21 in case of beta-cyclodextrin and 24 in case of gamma-cyclodextrin. In a given quantity of a cyclodextrin derivative, there will however generally be some cyclodextrin molecules that are not substituted at all, together with other molecules that have different numbers of quaternized ammonium substituents. A statistical average is therefore employed to characterize the number of quaternized ammonium groups of the entire quantity of cyclodextrin (molecules). The present invention embraces QACD compounds of formula (I) or (Ia) having an index n ranging from a value slightly above 0, for instance from about 0.1 to the theoretical upper values indicated above. This necessarily implies that the QACD derivatives may be used in the form of a mixture with other materials, such as unreacted cyclodextrin, and, as well, in substantially pure form. According to the art, the primary 6-position hydroxyl groups in any anhydroglucose group appear to be the most reactive, followed by the hydroxyl groups at the 2-position which are believed to be the next most reactive, and the hydroxyl at the 3-position as the least reactive. Irrespective of the actual sequence or order of reactions or the number of anhydroglucose units involved, the formulae (I) and (Ia) are intended to represent the QACD derivatives wherein the quaternized ammonium substitution may occur in different degrees of substitution at all or less than all anhydroglucose units in the cyclodextrin. Preferably n ranges from about 1 to 8, more preferably from about 1 to 4, for example from 1 to 3.

The QACD derivatives of formula (I) or (Ia), wherein the index h is 1, may, for instance, be prepared as described in detail in U.S.-Patent 3,453,257 or analogically. The compounds of formulae (I) and (Ia), wherein the index h is 0, may, for example, be prepared as described in U.S.-Patent 5,241,059.

Specific embodiments of compounds useful for the instant invention include the compounds of formula (Ia), wherein h is 1 and wherein R₁ is a branched or straight chain C₁-C₈alkylene or phenylene, which both may be substituted in one or more places; R₂, R₃ and R₄ are different or the same, and represent substituted or unsubstituted C₁-C₁₈alkyl; C₃-C₈cycloalkyl; phenyl; morpholinyl, pyridyl, pyrrolidyl, furfuryl, imidazolidyl or imidazolyl and X⁻ is a halide, nitrate, formate, acetate, butyrate, oleate, stearate, benzoate anion.

Particularly preferred is the use of compounds of formula (Ia), wherein R₁ is a branched or straight chain C₁-C₈alkylene, in particular C₁-C₄alkylene, more preferably ethylene or propylene, and R₂, R₃ and R₄ are different or the same, and are substituted or unsubstituted C₁-C₁₈alkyl, more particularly C₁-C₈alkyl, specifically C₁-C₄alkyl.

Preferred as well is the use of the compounds of formula (I) or (Ia), wherein the symbol 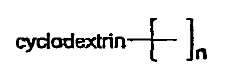 represents a n-valent residue of alfa-, beta- or gamma-cyclodextrin including the corresponding partially or fully etherified compounds.

Compounds of formula (I) and particularly of formula (Ia) can be used in an anti-infective pharmaceutical composition comprising said compounds of formula (1) and particularly of formula (1a) as described above.

Such pharmaceutical compositions are suitable for enteral, such as oral or rectal, parenteral, such as by intravenous, subcutaneous, intramuscular, or transdermal administration to mammals including humans, and useful for the treatment of infective disorders responsive thereto and comprise an anti-microbially elective amount of a QACD compound of formula (I) or preferably of formula (Ia) as the pharmacologically active compound, alone or in combination, with one or more pharmaceutically acceptable carriers and/or excipients suitable for either enteral or parenteral application.

Such compositions may, for instance, be in the form of tablets or gelatin capsules and comprise, for instance, one or more of the QACD compounds of formula (I) or (Ia) as active ingredient together with a) dituents, e.g. lactose, dextrose, sucrose, mannitol, sorbitol, cellulose and/or glycine; b) lubricants, e.g. silica, talcum, stearic acid, its magnesium or calcium salt and/or polyethyleneglycol; for tablets also c) binders e.g. magnesium aluminum silicate, starch paste, gelatin, tragacanth, methylcellulose, sodium carboxymethylcellulose and or polyvinylpyrrolidone: if desired d) disintegrants, e.g. starches, agar, alginic acid or its sodium salt, or effervescent mixtures; and/or e) absorbants, colorants, flavors and sweeteners. Injectable compositions according to the invention are preferably aqueous isotonic solutions or suspensions, and suppositories are advantageously prepared from fatty emulsions or suspensions. Said compositions may be sterilized, if desired, and contain adjuvants, such as stabilizing, wetting or emulsifying agents, salts for regulating the osmotic pressure and/or buffers. If desired, such compositions may also comprise additional preserving and/or solubilizing agents, although this is usually not necessary. Suitable formulations for transdermal application include an effective amount of **one** or more QACD compound of formula (I) or (Ia) with a carrier. Advantageous carriers include absorbable pharmacologically acceptable solvents to assist passage through the skin of the host. Characteristically, transdermal devices are in the form of a bandage comprising a backing member, a reservoir containing the compound optionally with carriers, optionally a rate controlling barrier to deliver the compound of the skin of the host at a controlled and predetermined rate over a prolonged period of time, and means to secure the device to the skin.

Other forms of such pharmaceutical compositions include inhalation formulations, emulsions, suspensions, rods, inserts, implants.

Suitable formulations for topical application, especially but not restricted to the skin and the eyes, include aqueous solutions, ointments, creams or gels well-known in the art. Compositions for topical use are particularly advantageous because the QACD compounds of formula (I) and (Ia) have unexpectedly been found to exhibit, in addition to their anti-microbial effect, firstly an excellent topical tolerability and secondly a particularly improved permeability through tissue like skin and especially ocular tissue, in particular corneal and/or conjunctival tissue.

Such pharmaceutical compositions may also contain other therapeutically valuable compounds and are generally prepared according to conventional mixing, granulating or coating methods, respectively, and contain about 0.1 to 75%, preferably about 1 to 50%, of the active ingredient

In a further aspect the instant invention relates to preserved pharmaceutical compositions comprising a preservative selected from the compounds of formula (I), and in particular from compounds of formula (Ia) and additionally one or more pharmaceutically active, preferably ophthalmic drug other than a compound of said formula (I) and (Ia). Such compositions comprise the compounds of formula (I) or (Ia) in preservatively effective amounts, for instance 0.01 to 10% based on the total weight of the composition, preferably 0.1 to 10%, e.g. 0-1 to 5%. If desired, said compositions may also comprise conventional preservatives in addition to the compounds of formulae (I) and (Ia), like for instance other quaternary ammonium compounds such as benzalkonium chloride (N-benzyl-N-(C₈-C₁₈alkyl)-N,N-dimethytammonium chloride), benzoxonium chloride or preservatives different from quaternary ammonium salts like alkyl-mercury salts:of thiosalicylic acid, such as, for example, thiomersal, phenylmercuric nitrate, phenylmercuric acetate or phenylmercuric borate, parabens, such as, for example, methylparaben or propylparaben, alcohols, such as, for example, chlorobutanol, benzyl alcohol or phenyl ethanol, guanidine derivatives, such as, for example, chlorohexidine or polyhexamethylene biguanide, sodium perborate, Germal^{®}II or sorbic add, in particular if the preservative efficacy of such combinations is increased when compared to the same amount of only one of the preservatives. A further specific embodiment of compositions according to the instant invention is free of such conventional preservatives.

A further unexpected advantage of the QACD compounds of formula (I) and (Ia) is their compatibility with aqueous solutions of hyaluronic acid compounds, in particular hyaluronic acid salts, e.g. sodium hyaluronate, which are a valuable and frequently used formulation component of pharmaceutical, in particular of ophthalmic compositions, however, are known to be incompatible with benzalkonium chloride, a very common preservative especially of ophthalmic compositions, together with which they irreversibly form a precipitate. The QACD compounds of formula (I) or formula (Ia) present an elegant way out of this dilemma because they provide an excellent preservative efficacy like benzalkonium chloride in combination with a good compatibility with hyaluronic acid derivatives which are incompatible with benzalkonium chloride.

As already mentioned above, the QACD compounds of formula (I) and (Ia) have furthermore proved to enhance the permeability of human or animal tissue, in particular of corresponding ocular and mucus tissue, for other drugs, specifically for drugs which form an inclusion complex with the respective QACD compounds. In a further aspect the present invention therefore relates to the use of compounds of formula (I) or (Ia) for enhancing the permeation of a drug through tissue and for enhancing the penetration of a drug into tissue, wherein said drug is preferably a drug typically administered topical to said tissue, and, in still a further aspect, to compositions comprising one or more pharmaceutical drug other than a compound of formula (I) or (Ia) and an enhancer for the permeability of said drug through or into the human or animal tissue, in particular ocular or mucus tissue, which enhancer is selected from the compounds of formula (I) and the compounds of formula (Ia) as described above.

A specific embodiment of said compositions does not comprise a zwitterionic drug in general, and/or specifically the drug ETH-615. described above.

Suitable drugs include but are not limited to anti-angiogenic drugs, anti-inflammatory drugs, anti-allergic drugs, anesthetic drugs, myopia preventing/inhibiting drugs, miotics, carbonic anhydrase inhibitors, alpha blocking agents, antioxidants, vitamins, and biologic materials like peptides, proteins, DNAs, RNAs and the like substances.

Specifically suitable drugs include furthermore ophthalmic and/or ocularly tolerable, which may, for example, be selected from the following drugs and combinations thereof:
- anti-inflammatory drugs, such as steroids, e.g. dexamethasone, fluorometholone, hydrocortisone, prednisolone; or so-called non-steroidal anti-inflammatory drugs (NSAID) such as COX-inhibitors, e.g. diclofenac, ketorolac, or indomethacin;
- anti-allergic drugs, selected e.g. from FK506, 33-epi-chloro-33-desoxy-ascomycin, cromolyn, emadine, ketotifen, levocabastine, lodoxamide, norketotifen, olopatadine, and rizabene;
- drugs to treat glaucoma (in particular intraocular pressure treatment), selected e.g. from latanoprost, 15-keto-latanoprost, unoprostone isopropyl, betaxolol, clonidine, levobunolol and timolol;
- anesthetic drugs, e.g. selected from cocaine hydrochloride, lidocaine, oxybuprocaine and tetracaine hydrochloride;
- myopia preventing/inhibiting drugs such as pirenzepine, atropine and the like;
- miotics, e.g. selected from carbachol, pilocarpine and physostigmine;
- carbonic anhydrase inhibitors, e.g. selected from acetazolamide and dorzolamide;
- alpha blocking agents, e.g. selected from apraclonidine and brimonidine; and
- antioxidants and/or vitamins, e.g. selected from ascorbic acid, retinol, retinol acetate, retinol palmitate, and natural and synthetic tocopherols, in particular alfa-tocopherol and alfa tocopherol acetate; and
- biologic materials like peptides, proteins, DNAs, RNAs and the like substances, and if desired from
- antifungal drugs, e.g. selected from amphotericin B, fluconazole and natamycin; and
- anti-viral drugs such as acyclovir, fomivirsen, ganciclovir, and trifluridine.

The addressed drugs are specifically useful for the topical treatment of a disease other than an infective disease, a fungus related disease, a viral disease, for example for the treatment of glaucoma, inflammation, allergy (such as hay fever), analgesia (e.g. surgery, mechanical ocular impacts, etc.) and the like. Particularly preferred ophthalmic drugs are therefore selected from anti-inflammatory drugs, anti-allergic drugs, and drugs to treat glaucoma.

It is known to those skilled in the art that a specific cyclodextrin compound does not automatically form an inclusion complex with any randomly chosen other compound, which may be desired. In such cases it is therefore preferred to use a QACD derivate derived from a cyclodextrin compound, for instance alfa-, beta- or gamma-cyclodextrin, that meets the cavity needs of the other component or components, e.g. the pharmaceutical drug/drugs.

The amount of compounds of formula (I) or formula (Ia) present in such an ophthalmic composition generally depends on the ophthalmic drug being used is typically in the range of 0.01 - 35%, preferably from 0.5 - 25%, e.g. from 5 -10%, 10 -15% and 15 - 20%. Also preferred are amount from 0.1 - 5% of the compound of formula (I) or (1a), in particular 0.5 - 5% and most particular 1-5% of said compound, by total weight of a corresponding ophthalmic composition.

The compositions for topical administration and/or ophthalmic compositions comprise advantageously a carrier suitable for topical administration, such as for example water, mixtures of water and water-miscible solvents, such as C₁-C₇-alkanols, vegetable oils or mineral oils comprising from 0.05 to 10%, preferably 0.5 to 5% by weight hydroxyethylcellulose, ethyl oleate, carboxymethylcellulose, and other non-toxic watersoluble polymers for ophthalmic uses, such as, for example, cellulose derivatives, such as methylcellulose, alkali metal salts of carboxymethylcellulose, hydroxymethylcellulose, methylhydroxypropylcellulose and hydroxypropylcellulose, acrylates or methacrylates, such as salts of polyacrylic acid or ethyl acrylate, polyacrylamides, natural products, such as gelatin, alginates, pectins, tragacanth, karaya gum, xanthan gum, carrageenin, agar and acacia, starch derivatives, such as starch acetate and hydroxypropyl starch, and also other synthetic products, such as polyvinyl alcohol, polyvinylpyrrolidone, polyvinyl methyl ether, polyethylene oxide, preferably cross-linked polyacrylic acid, such as neutral Carbopol, or mixtures of those polymers. Preferred carriers are water, cellulose derivatives, such as methylcellulose, alkali metal salts of carboxymethylcellulose, hydroxymethylcellulose, hydroxyethylcellulose, methylhydroxypropylcellulose and hydroxypropylcellulose, neutral Carbopol, or mixtures thereof. Highly preferred is water. The concentration of the carrier is, for example, from 1 to 100000 times the concentration of the active ingredient.

The ophthalmic compositions of the present invention may further comprise a tonicity enhancing agent. Tonicity enhancing agents are, for example, ionic compounds, such as boric acid, or alkali metal or alkaline earth metal halides, such as, for example, CaCl₂, KBr, KCl, LiCl, Nal, NaBr or NaCl. Non-ionic tonicity enhancing agents are, for example, urea, glycerol, sorbitol, mannitol, propylene glycol, or dextrose. For example, sufficient tonicity enhancing agent is added to impart to the ready-for-use ophthalmic composition an osmolality of approximately from 50 to 1000 mOsmol, preferred from 100 to 400 mOsmol, more preferred from 200 to 400 mOsmol and even more preferred from 280 to 350 mOsmol.

For the adjustment of the pH, preferably to a physiological pH, buffers may especially be useful. Examples of buffer substances are acetate, ascorbate, borate, hydrogen carbonate/carbonate, citrate, gluconate, lactate, phosphate, propionate and TRIS (tromethamine) buffers. Tromethamine and borate buffer are preferred buffers. The amount of buffer substance added is, typically, that necessary to ensure and maintain a physiologically tolerable pH range. The pH range is generally in the range of from 4 to 9, preferably from 4.5 to 8.5 and more preferably from 5.0 to 8.2.

Even though the compositions of the present invention may further comprise an additional preservative, e.g on storage or to inhibit microbial growth after opening a closed container holding such a composition and exposing such a composition to the air, this is normally not necessary because of the anti-microbial effect of the QACD compounds of formula (I) and (Ia). The term "anti-microbial effect" is meant to include an anti-bacterial, anti-fungal and anti-viral effect, in particular an anti-bacterial and an anti-fungal effect.

A composition of the present invention may additionally require the presence of a solubilizer, in particular if the active or the inactive ingredients tend to form a suspension or an emulsion.

A solubilizer suitable for compositions of the invention may, for example, be selected from the group consisting of tyloxapol, fatty acid glycerol polyethylene glycol esters, fatty acid polyethylene glycol esters, polyethylene glycols, glycerol ethers, other cyclodextrin compounds (for example alpha-, beta- or gamma-cyclodextrin, e.g. alkylated, hydroxyalkylated, carboxyalkylated or alkyloxycarbonyl-alkylated derivatives, or mono- or diglycosyl-alfa-, -beta- or gamma-cyclodextrin, mono- or dimaltosyl-alfa-, -beta- or -gamma-cyclodextrin or panosyl-cyclodextrin), polysorbate 20, polysorbate 80 or mixtures of those compounds. A specific example of an especially preferred solubilizer is a reaction product of castor oil and ethylene oxide, for example the commercial products Cremophor EL^{®}or Cremophor RH 40^{®}. Reaction products of castor oil and ethylene oxide appear to be particularly good solubilizers that are tolerated extremely well by the eye. Another preferred solubilizer is selected from tyloxapol and from a cyclodextrin. The concentration used depends especially on the concentration of the active ingredient. The amount added is typically sufficient to solubilize the active ingredient. For example, the concentration of the solubilizer is typically from 0.1 to 5000 times the concentration of the active ingredient.

A composition of the invention may comprise further non-toxic excipients, such as, for example, emulsifiers, wetting agents or fillers, such as, for example, polyethylene glycols, e.g. having an average molecular weight of 200, 300, 400 and 600, or higher polyethylene glycols, such as Carbowax 1000, 1500, 4000, 6000 and 10000. Other excipients that may be used if desired are listed below but are not intended to limit in any way the scope of the possible excipients. They are especially complexing agents, such as disodium-EDTA or EDTA, antioxidants, such as ascorbic acid, acetylcysteine, cysteine, sodium hydrogen sulfite, butyl-hydroxyanisole, butyl-hydroxytoluene or alpha-tocopherol acetate; stabilizers, such thiourea, thiosorbitol, sodium dioctyl sulfosuccinate or monothioglycerol; or other excipients, such as, for example, lauric acid sorbitol ester, triethanol amine oleate or palmitic acid ester. Preferred exipients are complexing agents, such as disodium-EDTA. The amount and type of excipient added is in accordance with the particular requirements and is generally in the range of from approximately 0.0001 to approximately 90% by weight.

A further problem frequently arises when a drug is topically administered, namely that it is often washed off again from the tissue to which it is applied during an unacceptably short time period. Compositions comprising one or more pharmaceutical drug, a compound of formula (I) or (Ia) as described hereinabove and a carrier comprising a polymer surprisingly provide a solution to this problem, too. It has namely been found that said compositions allow a sustained or prolonged drug delivery at the place of administration.

Therefore the present invention includes a further aspect, the use of a composition comprising one or more pharmaceutical drug, a compound of formula (I) or (Ia) and a carrier comprising a polymer as a drug dosage system for sustained delivery.

Compositions according to the instant invention which are especially suitable as a drug dosage system for sustained delivery are preferably in a semi-solid (paste-like) or more preferably a solid state and are, for example, in the form of a film, a rod, a bar, a capsule, a corneal shield, a corneal ring, an implant, an insert, an intra-ocular lens, a therapeutic contact lens, a tablet, e.g. a mini tablet, a mini-disc, or a pellet.

Carriers suitable for solid state medicaments representing a dosage system for sustained drug delivery according to the invention are for example selected from
- a matrix of a bioerodible polymer preferably being selected from the group consisting of polyhydroxy-acids, such as polylactic acid and polyglycolic acid; polyesters, polyorthoesters, polyanhydrides, polycyanoacrylates, natural gums, such as acacia gum and arabic gum; celluloses, such as carboxymethylcellulose; methacrylate (co)polymers such as Eudragits, e.g. Eudragit RL PO, Eudragit RS PO; and/or
- a bioadhesive polymer preferably being selected from the group consisting of maltodextrin, celluloses, such as carboxymethyl cellulose, hydroxyethyl cellulose; chitosans; hyaluronic acid; polyacrylates e.g. carbopol; polycarbophils e.g. Noveon AA-1; polyvinylalcohol such as Mowiol 26-88; polyvinylpyrrolidone such as povidone K30.

Accordingly, the invention relates to a solid state medicament selected from a film, a rod, a bar, a capsule, a corneal shield, a corneal ring, an implant, an insert, an intra-ocular lens, a therapeutic contact lens, a tablet, e.g. a mini tablet, a mini-disc, and a pellet comprising a pharmaceutically effective drug, a compound of formula (I) or (Ia) and a carrier suitable for the manufacure of a solid state medicament. Said solid state medicaments provide in particular the synergistic advantage of sustained drug delivery with improved drug permeability.

The physical properties/appearances of a solid state medicament according to the invention depends on the carriers used, and its physical appearance may, for example, range from soft to stiff, from water soluble up to water insoluble, from transparent to opaque and so on.

The concentration of an above carrier is, for example, from 1 to 100000 times the concentration of the active ingredient.

An even more specific aspect of the instant invention is an ophthalmic composition comprising a QACD compound of formula (I) or (Ia), at least a further pharmaceutically active component like an ophthalmic drug which is a novel drug delivery system providing the synergistic properties of improved drug delivery, improved tolerability and excellent preservative efficacy. Drug delivery as used herein refers particularly to topical ocular administration.

Still further aspects of the instant invention are
- a method for preserving a pharmaceutical composition comprising adding to said pharmaceutical composition an effective amount of a preservative selected from the compounds of formula (I); preferably selected from the compounds of formula (Ia);
- a method for enhancing the permeability of a drug contained in a pharmaceutical composition through skin, buccal, mucosal, pulmonal, vaginal or ocular, in particular conjunctival tissue, more particularly for enhancing the permeability of a drug contained in an ophthalmic composition through ocular tissue, especially the corneal permeation, comprising adding to said composition a compound selected from the compounds of formula (I), preferably selected from the compounds of formula (Ia);
- a method for synergistically enhancing the permeability of a drug contained in a pharmaceutical composition through skin, buccal, mucosal, pulmonal, vaginal or ocular, in particular conjunctival tissue, more particularly for enhancing the permeability of a drug contained in an ophthalmic composition through ocular tissue, especially the corneal permeation, and for preserving said composition comprising adding to said composition a compound selected from the compounds of formula (I), preferably selected from the compounds of formula (Ia); and
- a method for controlled, in particular, sustained or prolonged delivery of an organic compound, in particular a pharmaceutically active drug at the place of administration, comprising the following steps:
   mixing said drug at least with a compound selected from the compounds of formula (I) corresponding to claim 1, in particular selected from the compounds of formula (Ia) according to claim 2 and a polymer, in particular one or more of a bioerodible polymer and/or one or more of a bioadhesive polymer, and administering the composition obtained thereby.

### Example 1: Increase of corneal permeation

### Corneal permeation device:

The device used is a modified Valia-Chien system consisting of two water-jacketed cells for temperature control. Each cell is filled with GBR buffer (see below), stirred by a magnet and continuously gassed with Oxycarbon (5 % CO₂ / 95 % O₂), During an experiment, the cells are separated by the cornea, one cell containing the test substance dissolved in GBR and acting as donor (tear side), the other one being the acceptor (aqueous humor side).

### Corneas:

Pig eyes are obtained from the local abattoir. They are kept in Dulbecco's MEM (minimal essential medium) with Glutamax-I (Gibco) on ice and used within a few hours after receipt.

### Buffers:

Buffers for *in vitro* corneal permeation studies are adapted from glutathione-bicarbonate-Ringer (GBR) solution. "GBR aqueous humor" is used in the acceptor cell and "GBR tears" on the donor side for equilibration. Their composition is listed in Table 1..

### Assay of corneal permeation:

On receipt from the abattoir the eye is mounted on a dissection board, cornea facing up. After checking integrity of the cornea, the sclera is incised approximately 1-2 mm from the corneal rim with a scalpel and the anterior segment is excised. The iris and lens are carefully removed with forceps without damaging the corneal structures. The cornea is then mounted between the two cells of the permeation device with the help of a pinch clamp. Immediately, 3 ml of previously warmed and gassed GBR buffer are added to each cell, carefully removing any trapped air bubbles in the cells. The system is gassed and stirred for about 30 minutes at 35 °C. After equilibration, the donor side is emptied and the same amount of a previously warmed formulation of active substance is added at time t=0. An aliquot of 300 µl "GBR aqueous humor" is taken at time t=0 from the acceptor cell and the missing volume is replaced by the same volume of fresh buffer. Subsequently, this procedure is repeated in the acceptor cell at predefined time points and the aliquots are analysed for active by HPLC. Both compartments are kept under constant stirring with small magnets. The usual duration of an experiment is 180 minutes which is also the time of contact of the formulation with the cornea.

**Table 1: Buffers used for in vitro corneal permeation experiments**

| Constituent | GBR aqueous humor | GBR tears |
|---|---|---|
| | Concentration [mM] | Concentration [mM] |
| NaCl | 95.75 | 115.75 |
| NaH₂PO₄ | 1.25 | 1.25 |
| KCI | 4 | 20 |
| CaCl₂ | 2 | 2 |
| MgCl₂ | 1 | 1 |
| Adenosine | 0.5 | 0.5 |
| NaHCO₃ | 23 | 23 |
| Glutathione reduced | 0.3 | 0.3 |
| Glucose | 77.7 | 27.75 |
| H₂O | q.s. | q.s. |
| pH | 7.3-7.4* | 7.3-7.4* |
| Osmolality | 297 mOsm/kg | 311 mOsm/kg |

| | | |
|---|---|---|
| * when gassed with 5% CO₂ / 95% O₂ | | |

### A) Corneal permeation experiments with diclofenac formulations

### 1) Diclofenac sodium 0.1% without thiomersal (marketed Voltaren Ophtha formulation, SDU)

| Time (min) | Average permeated amount (micro-gram) | S.D. |
|---|---|---|
| 0 | 0 | 0 |
| 30 | 0 | 0 |
| 60 | 0 | 0 |
| 90 | 0.1970182 | 0.160121 |
| 120 | 0.5716975 | 0.385907 |
| 180 | 1.6826328 | 0.78374 |

### 2) Diclofenac sodium 0.1% with 2% HP-gamma-cyclodextrin and without BAC

| Time (min) | Average permeated amount (micro-gram) | S.D. |
|---|---|---|
| 0 | 0 | 0 |
| 30 | 0 | 0 |
| 60 | 0.2512672 | 0.237461 |
| 90 | 1.2153835 | 0.532895 |
| 120 | 2.2474393 | 0.707873 |
| 180 | 6.4313489 | 1.643572 |

### 3) Diclofenac sodium 0.1 % with 0.1 % QA-beta-CD and without BAC

| Time (min) | Average permeated amount (micro-gram) | S.D. |
|---|---|---|
| 0 | 0 | 0 |
| 30 | 0.263057 | 0.15532 |
| 60 | 1.767697 | 1.093765 |
| 90 | 5.807919 | 1.235891 |
| 120 | 10.71147 | 2.608262 |
| 180 | 20.08034 | 3.651529 |

BAC = benzalkonium chloride
HP-gamma-cyclodextrin = hydroxypropyl-gamma-cyclodextrin
QA-beta-CD = 3-(trimethylammonio)-2-hydroxypropyl-beta-cyclodextrin chloride

In the above experiments the efficacy in drug permeation an embodiment of this invention (QA-beta-CD [item 3)]) can directly be compared with respect to the prior art situation (HP-gamma-cyclodextrin; [item 2)]).

### B) Corneal permeation experiments with 5-methyl-2-(2'chloro-6'-fluoroanilino)phenyl acetic acid (Compound B)

### 1) Compound B 0.1% without thiomersal (analogous to marketed Voltaren Ophtha formulation, SDU)

| Time (min) | Average permeated amount (micro-gram) | S.D. |
|---|---|---|
| 0 | 0 | 0 |
| 30 | 0 | 0 |
| 60 | 0.0840 | 0.1361 |
| 90 | 0.4359 | 0.2176 |
| 120 | 0.8800 | 0.3568 |
| 180 | 2.0137 | 0.4391 |

### 2) Compound B 0.1% with 2% HP-gamma-cyclodextrin and without BAC

| Time (min) | Average permeated amount (micro-gram) | S.D. |
|---|---|---|
| 0 | 0 | 0 |
| 30 | 0 | 0 |
| 60 | 0.9456 | 0.7494 |
| 90 | 3.6156 | 2.1221 |
| 120 | 5.5475 | 2.8703 |
| 180 | 10.4593 | 4.4961 |

### 3) Compound B 0.1 % with 0.1 % QA-beta-cyclodextrin and without BAC

| Time (min) | Average permeated amount (micro-gram) | S.D. |
|---|---|---|
| 0 | 0 | 0 |
| 30 | 0 | 0 |
| 60 | 2.2883 | 1.4595 |
| 90 | 6.7793 | 2.8701 |
| 120 | 12.4568 | 2.7525 |
| 180 | 21.9144 | 5.1823 |

BAC = benzalkonium chloride
HP-gamma-cyclodextrin = hydroxypropyl-gamma-cyclodextrin
QA-beta-cyclodextrin = 3-(trimethylammonio)-2-hydroxypropyl-beta-cyclodextrin chloride, empiric formula: (C₆H₁₀₋ₙO₅)₇(C₆H₁₅ONCl)ₙ n= 2-5.

Again, the efficacy in drug permeation of an embodiment of this invention (QA-beta-CD [item 3)]) can directly be compared with respect to the prior art situation (HP-gamma-cyclodextrin; [item 2)]), in these experiments.

### Example 2: Preservative effectiveness test

0.2 g of QA-beta-CD (QA-beta-CD = 3-(trimethylammonio)-2-hydroxypropyl-beta-cyclodextrin chloride, empiric formula: (C₆H₁₀₋ₙO₅)₇(C₆H₁₅ONCl)ₙ n= 2-5. are added to 170 ml water (nanopure). Then 10.10 g of Sorbitol and 0.2 g of Disodium edetate are added and the pH is adjusted to a value of 7.0-7.4 and water added to make up 200 ml of resulting solution. The solution is filtered through a Corning bottle top filter unit 0.22 µm CA under sterile conditions. The solution has a pH of 7.35 and an osmolality of 307 mOsm/kg (270-330)

Samples of the solution are inoculated with Escherichia coli ATCC 8739; Pseudomonas aeruginosa ATCC 9027, Staphyloccocus aureus ATCC 6538; Candida albicans ATCC 10231 and Aspergillus niger ATCC 16404, respectively and assayed for presence/growth at the times given in Table 2.

**Table 2:**

| | Escherichia coli ATCC 8739 | Pseudomonas aeruginosa ATCC 9027 | Staphyloccocus aureus ATCC 6538 | Candida albicans ATCC 10231 | Aspergillus niger ATCC 16404 |
|---|---|---|---|---|---|
| Inoculum | 2.5 10⁶ | 2.0 10⁶ | 1.1 10⁶ | 1.1 10⁶ | 2.9 10⁵ |
| Time 0h | 2.5 10⁵ | 2.2 10⁵ | 1.5 10⁵ | 1.4 10⁵ | 1.1 10⁴ |
| Time 6h | <10² | <10² | 5.4 10³ | - | - |
| Time 24h | <10² | <10² | <10² | - | - |
| Time 7d | <10² | <10² | <10² | <10² | 2.7 10³ |
| Time 14d | - | - | - | <10² | 2 10² |
| Time 21d - | | - | - | - | - |
| Time 28 d | <10² | <10² | <10² | <10² | 2. 10² |

The tested solution meets the requirements as defined as European Pharmacopeia (Eur. Ph.) criteria A for ophthalmic preparations, e.g. as described in Eur. Ph. Supplement 2001, Section 5.1.3, page 293 to 295.

### Example 3:

Films able to act as ocular insert are prepared and tested. The compositions of the films are disclosed in Table 3.
All preparations are made under magnetic stirring (400rpm) and at room temperature. Firstly, the glycerol is dissolved in 5ml of double distilled water or, alternatively, in 5ml of aqueous phosphate buffer (pH=7) for the composition A and B. Then the Mowiol 26-88 is added to said water or said phosphate buffer comprising said glycerol until complete dissolution. The D-mannitol or QA-beta-CD are dissolved in the resulting solution and after complete dissolution of these components, the polyvinylpyrrolidone is added to the mixture. At last, ketotifen hydrogenfumarate is added under stirring, and the resulting solutions are then centrifuged (500 x g, 25min, 25°C) in order to remove impurities. A II solutions were very clear.
The solutions are then cast in petri dishes and dried in a desiccator (containing phosphor pentoxyde) under vacuum (13mbar). Upon drying, the resulting thin layer films are scored according the semi quantitative scale of Table 4. Two individuals conducted said evaluation blindly and independently.

Table 5 shows the mean value of the overall score (possible range 0 to 9) of the thin layer films cast in the Petri dishes.

After drying, the preparations comprising Mowiol 26-88, glycerol and QA-beta-CD give excellent results. The maximum score is obtained for the basic preparation loaded with QA-beta-CD (E). This score is significantly higher than the one obtained when the preparation is loaded with D-mannitol (B). The resulting thin layer film is highly transparent, easy to remove from the cast support and it exhibits a regular aspect and is essentially homogeneous.
The addition of polyvinylpyrrolidone in the preparation is efficient and the resulting films exhibit good scores. This is particularly the case when the preparations are loaded with QA-beta-CD (D).
Furthermore the compositions F to K are prepared, analogous to the compositions A and D but loaded with different amounts of ketotifen hydrogen fumarate (Zaditen), namely 6.75, 12.5 and 20% respectively. The resulting layers evidence good and equivalent scores with 6.75 and 12.5% of drug (F and I and G and J respectively). When the films are loaded with 40 mg (20%) of Zaditen (H, K), the presence of some crystalline structures is found in the film based on D-mannitol. This re-crystallization does not occur in presence of QA-beta-CD, probably because of a certain interaction between the QACD component and the drug.

Appropriate thin layer films are obtained with the addressed compositions containing QA-beta-CD.

**Table 3: Thin layer film composition (in mg. per 200mg film)**

| Composition | A | B | C | D | E | F | G | H | I | J | K |
|---|---|---|---|---|---|---|---|---|---|---|---|
| Mowiol 26-88 | 100 | 140 | 100 | 100 | 140 | 100 | 100 | 100 | 100 | 100 | 100 |
| Hydroxypropyl cellulose | | | 40 | | | | | | | | |
| Polyvinylpyrrolidone | 40 | - | | 40 | - | 40 | 40 | 40 | 40 | 40 | 40 |
| D-mannitol | 50 | 50 | | | | 36.5 | 25 | 10 | | | |
| QA-beta-CD | | | 50 | 50 | 50 | | | | 36.5 | 25 | 10 |
| Glycerin | 10 | 10 | 10 | 10 | 10 | 10 | 10 | 10 | 10 | 10 | 10 |
| Ketotifen hydrogen fumarate | | | | | | 13.5 | 25 | 40 | 13.5 | 25 | 40 |

**Table 4: Semi quantitative scale used to score the thin layer films**

| Criterion | Observation | Score |
|---|---|---|
| Thin layer film formed | Yes | 1 |
| | No | 0 |
| Transparency | Good | 3 |
| | Average | 2 |
| | Poor | 1 |
| | None | 0 |
| Surface aspect | Regular | 1 |
| | Non-regular | 0 |
| Homogeneity | Yes | 1 |
| | No | 0 |
| Remove from cast support | Easy | 3 |
| | Average | 2 |
| | Difficult | 1 |
| | None | 0 |

**Table 5:**

| Composition | Macroscopic Score |
|---|---|
| A | 7.5 |
| B | 7.5 |
| C | 5.5 |
| D | 8 |
| E | 9 |
| F | 7.5 |
| G | 8 |
| H | 8 |
| I | 7.5 |
| J | 8.5 |
| K | 9 |

## Claims

1. Use of a compound of formula (I): 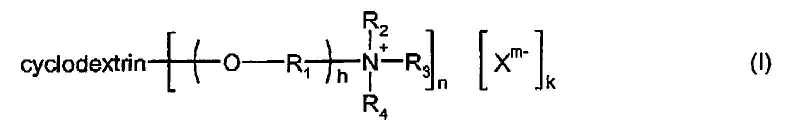 wherein the symbol 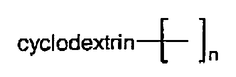 represents a n-valent residue derived from a cyclodextrin compound by removing n of its hydroxyl groups;
n is a number greater than 0 and represents the average number of substituents of formula 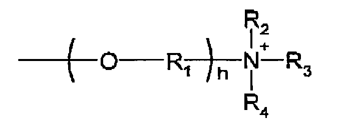 per molecule of said compound;
h is 0 or 1:
R₁ is a di-valent group selected from alkylene, hydroxy alkylene, halogeno alkylene, monocyclic aralkylene, cycloalkylene and phenylene;
R₂, R₃ and R₄ are each independently of one another a group selected from alkyl, cycloalkyl, aryl, aralkyl, and cycloheteryl;
X^{m-} is a m-fold negatively charged anion;
m is an integer being equal or greater than 1; and k is n/m
in the preparation of an anti-infective medicament.

2. Use according to claim 1 of a compound of formula (Ia): 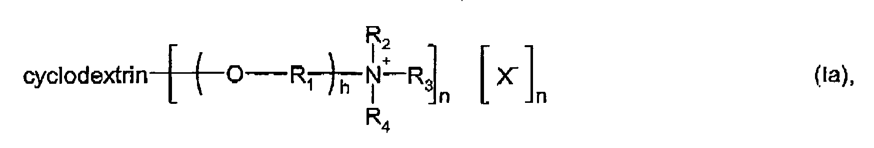 wherein X⁻ is a simple-charged anion and the other residues and h and n have the meaning as in formula (I).

3. Use according to claim 2, wherein
R₁ is a branched or straight chain C₁-C₈alkylene or phenylene, which both may be substituted in one or more places; and
R₂, R₃ and R₄ are different or the same, and are substituted or unsubstituted C₁-C₁₈alkyl; C₃-C₆cycloalkyl; phenyl; morpholinyl, pyridyl, pyrrolidyl, furfuryl, imidazolidyl or imidazolyl and
X⁻ is a halide, nitrate, formate, acetate, butyrate, oleate, stearate, benzoate anion.

4. Use according to claim 3, wherein
R₂, R₃ and R₄ are different or the same, and are substituted or unsubstituted C₁-C₁₈alkyl.

5. Use according to claim 4, wherein
R₁ is a branched or straight chain C₁-C₈alkylene and
R₂, R₃ and R₄ are different or the same, and are substituted or unsubstituted C₁-C₈alkyl.

6. Use according to claim 5, wherein
R₁ is a branched or straight chain C₁-C₄alkylene and
R₂, R₃ and R₄ are different or the same, and are unsubstituted C₁-C₄alkyl.

7. Use of a compound according to anyone of claims 1 to 5, wherein the symbol 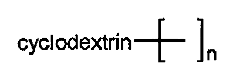 represents a residue of alfa-, beta- or gamma-cyclodextrin or a derivative of said cyclodextrins.

8. Use according to anyone of claims 1 to 7, wherein n is 0.1 to 24, preferably about 1 to 8.

9. Use according to anyone of claims 1 to 8, wherein h is 1.

10. Use according to anyone of claims 1 to 8, wherein h is 0.

11. A pharmaceutical composition comprising a preservative selected from the compounds of formula (I) corresponding to claim 1, in particular selected from the compounds of formula (Ia) according to claim 2; and additionally one or more pharmaceutically active drugs other than a compound of formula (I) or (Ia).

12. A composition according to claim 11, wherein the preservative is present in a concentration of 0.01 to 10% by weight of the total composition.

13. An ophthalmic composition according to claim 11 or 12 comprising one or more ophthalmic drug.

14. An ophthalmic composition comprising one or more ophthalmic drug and an enhancer for the permeability of said drug through ocular tissue, in particular for the corneal permeation of said drug, selected from the compounds of formula (I) corresponding to claim 1, in particular selected from the compounds of formula (Ia) according to claim 2.

15. A composition according to claim 14, wherein the enhancer is present in a concentration ranging from 0.01 - 35%, preferably from 0-5 - 25%, more preferably from 5 - 10%, and 15 - 20%, also preferably from 0.1-5%, 0.5 - 5% and 1-5% by total weight of the ophthalmic composition.

16. A composition according to any of claims 13 to 15 comprising one or more ophthalmic drug selected from:
- anti-inflammatory drugs selected from steroids and non-steroidal anti-inf ammatory drugs (NSAID);
- anti-allergic drugs, in particular selected from FK506, 33-epi-chloro-33-desoxy-ascomycin, cromolyn, emadine, ketotifen, levocabastine, Iodoxamide, norketotifen, olopatadine, and rizabene;
- drugs to treat glaucoma (in particular intraocular pressure treatment), in particular selected from latanoprost, 15-keto-latanoprost, unoprostone isopropyl, betaxolol, clonidine, levobunolol and timolol;
- anesthetic drugs, in particular selected from cocaine hydrochloride, lidocaine, oxybuprocaine and tetracaine hydrochloride;
- myopia preventing/inhibiting drugs;
- miotics, in particular selected from carbachol, pilocarpine and physostigmine;
- carbonic anhydrase inhibitors, in particular selected from acetazolamide and dorzolamide;
- alpha blocking agents, in particular selected from apraclonidine and brimonidine; and
- antioxidants and/or vitamins, in particular selected from ascorbic acid, retinol, retinol acetate, retinol palmitate and natural and synthetic tocopherols, in particular alfa-tocopherol and alfa tocopherol acetate; and
- biologic materials, in particular peptides, proteins, DNAs or RNAs.

17. A composition according to claim 16 wherein the ophthalmic drug is selected from anti-inflammatory drugs, anti-allergic drugs, and drugs to treat glaucoma.

18. A composition according to any of claims 13 to 17, comprising one or more ophthalmically acceptable excipients.

19. A composition according to claim 18 wherein the excipients comprise a salt of hyaluronic acid.

20. A composition according to claim 19, which is substantially free of benzalkonium chloride.

21. Use of a composition comprising one or more pharmaceutically active drug, a compound of formula (I) and a carrier comprising a polymer as a drug dosage system for sustained delivery.

22. Use according to claim 21, wherein the drug dosage system for sustained delivery is in a semi-solid (paste-like) or, preferably, in a solid state.

23. Use according to claim 21 or 22, wherein the carrier is a matrix of one or more of a bioerodible polymer and/or one or more of a bioadhesive polymer.

24. Use according to claim 23, wherein the bioerodible polymer is selected from polyhydroxy-acids, in particular polylactic acid and polyglycolic acid; polyesters, polyorthoesters, polyanhydrides, polycyanoacrylates, natural gums, in particular acacia gum and arabic gum; celluloses; and (meth)acrylate (co)polymers.

25. Use according to claim 23 or preferably 24, wherein the bioadhesive polymer is selected from maltodextrin ; celluloses; chitosans; hyaluronic acid; polyacrylates; polycarbophils; polyvinylalcohols and polyvinylpyrrolidones.

26. A drug dosage system for sustained delivery essentially consisting of a composition comprising one or more pharmaceutically active drug, a compound of formula (I) and a carrier comprising a polymer and selected from a film, a rod, a bar, a capsule, a corneal shield, a corneal ring, an implant, an insert, an intra-ocular lens, a therapeutic contact lens, a tablet, mini tablet, a mini-disc, and a pellet.

27. A method for preserving a pharmaceutical composition comprising adding to said pharmaceutical composition an effective amount of a preservative selected from the compounds of formula (I) corresponding to claim 1; preferably selected from the compounds of formula (Ia) corresponding to claim 2.

28. Use of a compound selected from the compounds of formula (I) corresponding to claim 1, in particular selected from the compounds of formula (Ia) according to claim 2, in the preparation of a pharmaceutical composition for enhancing the permeability of a drug contained in said pharmaceutical composition through skin, buccal, mucosal, pulmonal, vaginal or ocular, in particular conjunctival tissue, more particularly for enhancing the permeability of a drug contained in an ophthalmic composition through ocular tissue, especially the corneal tissue..

29. Use of claim 28 wherein said enhancement is a synergistic enhancement.

## Patentansprüche

1. Verwendung einer Verbindung der Formel (I) 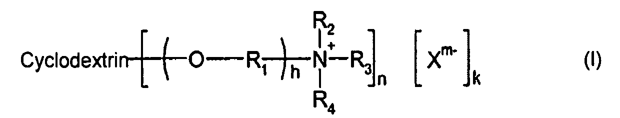 worin das Symbol 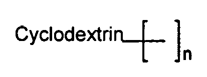 für einen n-valenten Rest steht, der abgeleitet ist von einer Cyclodextrinverbindung durch Entfernung von n an ihren Hydroxylgruppen,
wobei n eine Zahl von größer als 0 ist und den Mittelwert an Substituenten der Formel 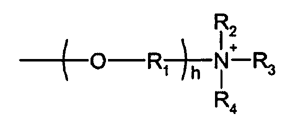 pro Molekül dieser Verbindung repräsentiert,
h für 0 oder 1 steht,
R₁ eine divalente Gruppe ist, die ausgewählt ist aus Alkylen, Hydroxyalkylen, Halogenalkylen, monocyclischem Aralkylen, Cycloalkylen und Phenylen,
R₂, R₃ und R₄ jeweils unabhängig voneinander für eine Gruppe stehen, die ausgewählt ist aus Alkyl, Cycloalkyl, Aryl, Aralkyl und Cycloheteroyl,
X^{m-} für ein m-fach negativ geladenes Anion steht,
m für eine Zahl steht, die gleich oder größer ist als 1, und
k für n/m steht,
bei der Herstellung eines antiinfektiven Arzneimittels.

2. Verwendung nach Anspruch 1 einer Verbindung der Formel (Ia) 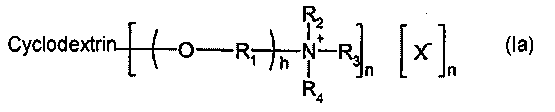 worin X⁻ für ein einfach geladenes Anion steht und die anderen Reste, sowie h und n die in der Formel (I) angegebenen Bedeutungen haben.

3. Verwendung nach Anspruch 2, worin
R₁ für ein verzweigtes oder gerades C₁-C₈-Alkylen oder Phenylen steht, wobei beide an ein oder mehr Stellen substituiert sein können,
R₂, R₃ und R₄ gleich oder verschieden sind und für unsubstituiertes oder substituiertes C₁-C₁₈-Alkyl, C₃-C₆-Cycloalkyl, Phenyl, Morpholinyl, Pyridyl, Pyrrolidyl, Furfuryl, Imidazolidyl oder Imidazolyl stehen, und X⁻ ein Halogenid-, Nitrat-, Formiat-, Acetat-, Butyrat-, Oleat-, Stearat- oder Benzoatanion ist.

4. Verwendung nach Anspruch 3, worin
R₂, R₃ und R₄ gleich oder verschieden sind und für substituiertes oder unsubstituiertes C₁-C₁₈-Alkyl stehen.

5. Verwendung nach Anspruch 4, worin
R₁ für ein verzweigtes oder gerades C₁-C₈-Alkylen steht, und
R₂, R₃ und R₄ gleich oder verschieden sind und für substituiertes oder unsubstituiertes C₁-C₈-Alkyl stehen.

6. Verwendung nach Anspruch 5, worin
R₁ für ein verzweigtes oder gerades C₁-C₄-Alkylen steht, und
R₂, R₃ und R₄ gleich oder verschieden sind und für unsubstituiertes C₁-C₄-Alkyl stehen.

7. Verwendung einer Verbindung nach einem der Ansprüche 1 bis 5, worin das Symbol 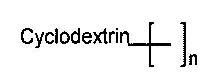 für einen Rest von alpha-, beta- oder gamma-Cyclodextrin oder ein Derivat eines solchen Cyclodextrins steht.

8. Verwendung nach einem der Ansprüche 1 bis 7, worin n für 0,1 bis 24, vorzugsweise für etwa 1 bis 8, steht.

9. Verwendung nach einem der Ansprüche 1 bis 8, worin h für 1 steht.

10. Verwendung nach einem der Ansprüche 1 bis 8, worin h für 0 steht.

11. Pharmazeutische Zusammensetzung, umfassend ein Konservierungsmittel, das ausgewählt ist aus den Verbindungen der Formel (I) entsprechend Anspruch 1, und das insbesondere ausgewählt ist aus den Verbindungen der Formel (Ia) entsprechend Anspruch 2, und zusätzlich ein oder mehr pharmazeutische Wirkstoffe, die keine Verbindungen der Formeln (I) oder (Ia) sind.

12. Zusammensetzung nach Anspruch 11, worin das Konservierungsmittel in einer Konzentration von 0,01 bis 10 Gew.-% der gesamten Zusammensetzung vorhanden ist.

13. Ophthalmische Zusammensetzung nach Anspruch 11 oder 12, umfassend ein oder mehr ophthalmische Wirkstoffe.

14. Ophthalmische Zusammensetzung, umfassend ein oder mehr ophthalmische Wirkstoffe und ein Mittel zur Verbesserung der Permeabilität eines solchen Wirkstoffs durch Augengewebe, besonders zur Permeation der Cornea durch einen solchen Wirkstoff, der aus den Verbindungen der Formel (I) entsprechend Anspruch 1 und besonders aus den Verbindungen der Formel (Ia) entsprechend Anspruch 2 ausgewählt ist.

15. Zusammensetzung nach Anspruch 14, worin das Mittel zur Verbesserung der Permeabilität in einer Konzentration im Bereich von 0,01 bis 35 %, vorzugsweise von 0,5 bis 25 %, bevorzugter von 5 bis 10 % und von 15 bis 20 %, und ferner bevorzugt von 0,1 bis 5 %, 0,5 bis 5 % und 1 bis 5 % vorhanden ist, bezogen auf das Gesamtgewicht der ophthalmischen Zusammensetzung.

16. Zusammensetzung nach einem der Ansprüche 13 bis 15, umfassend ein oder mehr ophthalmische Wirkstoffe, die ausgewählt sind aus
- antiinflammatorischen Wirkstoffen, die ausgewählt sind aus Steroiden und nicht steroidalen antiinflammatorischen Wirkstoffen (NSAID),
- antiallergischen Wirkstoffen, die besonders ausgewählt sind aus FK506, 33-Epichlor-33-desoxyascomycin, Cromolyn, Emadin, Ketotifen, Levocabastin, lodoxamid, Norketotifen, Clopatadin und Rizaben,
- Wirkstoffen zur Behandlung von Glaukom, besonders zur Behandlung eines Augeninnendrucks, die besonders ausgewählt sind aus Latanoprost, 15-Ketolatanoprost, Unoprostonisopropyl, Betaxolol, Clonidin, Levobunolol und Timolol,
- anesthetische Wirkstoffe, die besonders ausgewählt sind aus Cocainhydrochlorid, Lidocain, Oxybuprocain und Tetracainhydrochlorid,
- Wirkstoffe zur Prävention oder Inhibition von Myopie,
- Miotika, die besonders ausgewählt sind aus Carbachol, Pilocarpin und Physostigmin,
- Carboanhydraseinhibitoren, die besonders ausgewählt sind aus Acetazolamid und Dorzolamid,
- alpha-Blocker, die besonders ausgewählt sind aus Apraclonidin und Brimonidin, und
- Antioxidantien und/oder Vitamine, die besonders ausgewählt sind aus Ascorbinsäure, Retinol, Retinolacetat, Retinolpalmitat und natürlichen oder synthetischen Tocopherolen, besonders alpha-Tocopherol und alpha-Tocopherolacetat, und
- biologische Materialien, besonders Peptide, Proteine, DNAs und RNAs.

17. Zusammensetzung nach Anspruch 16, worin der ophthalmische Wirkstoff ausgewählt ist aus antiinflammatorischen Wirkstoffen, antiallergischen Wirkstoffen und Wirkstoffen zur Behandlung von Glaukom.

18. Zusammensetzung nach einem der Ansprüche 13 bis 17, umfassend ein oder mehr ophthalmisch akzeptable Exzipientien.

19. Zusammensetzung nach Anspruch 18, worin die Exzipientien ein Salz von Hyaluronsäure enthalten.

20. Zusammensetzung nach Anspruch 19, die praktisch frei von Benzalkoniumchlorid ist.

21. Verwendung einer Zusammensetzung, umfassend ein oder mehr pharmazeutische Wirkstoffe, eine Verbindung der Formel (I) und einen Träger, der ein Polymer umfasst, als ein Wirkstoffdosierungssystem für eine verzögerte Freigabe des Wirkstoffs.

22. Verwendung nach Anspruch 21, worin das Wirkstoffdosierungssystem für eine verzögerte Freigabe des Wirkstoffs in einem halbfesten (pastenartigen) oder vorzugsweise einem festen Zustand vorliegt.

23. Verwendung nach Anspruch 21 oder 22, worin der Träger eine Matrix aus ein oder mehr bioerodierbaren Polymeren und/oder ein oder mehr bioadhäsiven Polymeren ist.

24. Verwendung nach Anspruch 23, worin das bioerodierbare Polymer ausgewählt ist aus Polyhydroxysäuren, besonders Polymilchsäure und Polyglycolsäure, Polyestern, Polyorthoestern, Polyanhydriden, Polycyanoacrylaten, Naturgummis, besonders Akaziengummi und Gummi arabicum, Cellulosen und (Meth)acrylat(co)polymeren.

25. Verwendung nach Anspruch 23 oder vorzugsweise 24, worin das bioadhäsive Polymer ausgewählt ist aus Maltodextrin, Cellulosen, Chitosanen, Hyaluronsäure, Polyacrylaten, Polycarbophilen, Polyvinylalkoholen und Polyvinylpyrrolidonen.

26. Dosierungssystem für eine verzögerte Freigabe des Wirkstoffs, das im Wesentlichen besteht aus einer Zusammensetzung, die ein oder mehr pharmazeutische Wirkstoffe umfasst, einer Verbindung der Formel (I) und einem Träger, der ein Polymer umfasst und ausgewählt ist aus einem Film, einem Stäbchen, einer Barriere, einer Kapsel, einem Schutzfilm für die Kornea, einem Cornealring, einem Implantat, einem Einsatz, einer Intraokularlinse, einer therapeutischen Kontaktlinse, einer Tablette, einer Minitablette, einer Minischeibe und einem Pellet.

27. Verfahren zur Konservierung einer pharmazeutischen Zusammensetzung, durch Zusatz einer wirksamen Menge eines Konservierungsmittels zu einer solchen pharmazeutischen Zusammensetzung, das ausgewählt ist aus den Verbindungen der Formel (I) nach Anspruch 1 und vorzugsweise ausgewählt ist aus den Verbindungen der Formel (Ia) nach Anspruch 2.

28. Verwendung einer Verbindung, die ausgewählt ist aus den Verbindungen der Formel (I) nach Anspruch 1, und die besonders ausgewählt ist aus den Verbindungen der Formel (Ia) nach Anspruch 2, zur Herstellung einer pharmazeutischen Zusammensetzung für eine Verbesserung der Permeabilität eines Wirkstoffs, der in einer solchen pharmazeutischen Zusammensetzung enthalten ist, durch Haut-, Bukkal-, Mucosal-, Pulmonal-, Vaginal- oder Okulargewebe, besonders durch Bindegewebe, und speziell zur Verbesserung der Permeabilität eines Arzneimittels, das in einer ophthalmischen Zusammensetzung enthalten ist, durch Okulargewebe, besonders das Corneagewebe.

29. Verwendung nach Anspruch 28, worin die Verbesserung eine synergistische Verbesserung ist.

## Revendications

1. Utilisation d'un composé de formule (I): 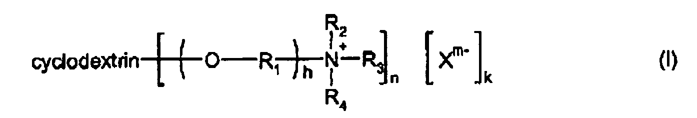 dans laquelle, le symbole 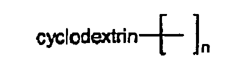 représente un résidu n-valent dérivé d'un composé cyclodextrine suite à l'extraction de n des groupes hydroxyles ;
n est un nombre supérieur à 0 et représente le nombre moyen de substituants de formule 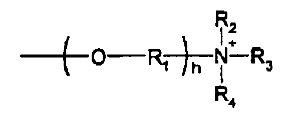 par molécule dudit composé ;
h est 0 ou 1 ;
R₁ est un groupe di-valent sélectionné à partir d'alkylène, hydroxy alkylène, halogéno alkylène, aralkylène monocyclique, cycloalkylène et phénylène ;
R₂, R₃ et R₄ sont, indépendamment les uns des autres, un groupe sélectionné à partir d'alkyle, cycloalkyle, aryle, aralkyle et cyclohétéryle ;
X^{m-} est un anion chargé négativement m-fois ;
m est un nombre entier égal ou supérieur à 1 : et
k est n/m
dans la préparation d'un médicament anti-infectieux.

2. Utilisation selon la revendication 1 d'un composé de formule (Ia) : 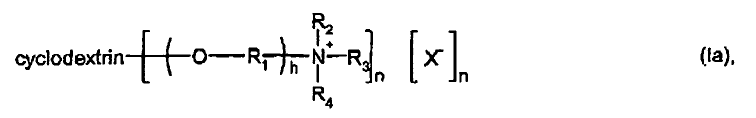 dans laquelle, X⁻ est un anion simple chargé et dans laquelle les autres résidus et h et n possèdent la signification donnée à la formule (I).

3. Utilisation selon la revendication 2, **caractérisée en ce que**
R₁ est un C₁-C₈alkylène ou phénylène à chaîne linéaire ou ramifiée, tous deux pouvant être substitué au niveau d'une ou plusieurs places ; et
R₂, R₃ et R₄ sont différents ou identiques, et sont C₁-C₆alkyle substitué ou non substitué ; C₃-C₆cycloalkyle ; phényle ; morpholinyle, pyridyle, pyrrolidyle, furfuryle, imidazoliclyle ou imidazolyle et
X⁻ est un anion halogénure, nitrate, formate, acétate, butyrate, aléate, stéarate, benzoate.

4. Utilisation selon la revendication 3, **caractérisée en ce que**
R₂, R₃ et R₄ sont différents ou identiques, et sont C₁-C₁₈alkyle substitué ou non substitué.

5. Utilisation selon la revendication 4, **caractérisée en ce que**
R₁ est un C₁-C₈alkylène à chaîne linéaire ou ramifiée ; et
R₂, R₃ et R₄ sont différents ou identiques, et sont C₁-C₈alkyle substitué ou non substitué.

6. Utilisation selon la revendication 5, **caractérisée en ce que**
R₁ est un C₁-C₄alkylène à chaîne linéaire ou ramifiée ; et
R₂, R₃ et R₄ sont différents ou identiques, et sont C₁-C₄alkyle non substitué.

7. Utilisation d'un composé selon l'une quelconque des revendications 1 à 5, **caractérisée en ce que** le symbole 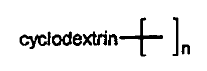 représente un résidu d'alfa-, bêta- ou gamma-cyclodextrins ou un dérivé desdites cyclodextrine.

8. Utilisation d'un composé selon l'une quelconque des revendications 1 à 7, **caractérisée en ce que** n est compris dans la gamme allant d'environ 0,1 à 24, de préférence d'environ 1 à 8.

9. Utilisation d'un composé selon l'une quelconque des revendications 1 à 8, **caractérisée en ce que** h est 1.

10. Utilisation d'un composé selon l'une quelconque des revendications 1 à 8, **caractérisée en ce que** h est 0.

11. Composition pharmaceutique comprenant un conservateur sélectionné à partir des composés de formule (I) correspondant à la revendication 1, plus particulièrement sélectionné à partir des composés de formule (Ia) selon la revendication 2 ; et comprenant également un ou plusieurs produits pharmaceutiquement actifs autres qu'un composé de formule (I) ou (Ia).

12. Composition selon la revendication 11, **caractérisée en ce que** le conservateur est présent selon une concentration comprise dans la gamme allant de 0,01 à 10% en poids de la composition totale.

13. Composition ophtalmique selon l'une quelconque des revendications 11 ou 12 comprenant un ou plusieurs produits ophtalmiques.

14. Composition ophtalmique comprenant un ou plusieurs produits ophtalmiques et une substance permettant d'accroître la stabilité dudit produit par l'intermédiaire du tissu oculaire, tout particulièrement en vue de la perméation de la cornée dudit produit, sélectionnés à partir des composés de formule (I) correspondant à la revendication 1, plus particulièrement sélectionné à partir des composés de formule (Ia) selon la revendication 2.

15. Composition selon la revendication 14, **caractérisée en ce que** la substance permettant d'accroître la stabilité est présente selon une concentration comprise dans la gamme allant de 0,01 - 35%, de préférence de 0,5 - 25%, de préférence encore de 5 - 10%, et de 15 - 20%, et de préférence encore de 0,1 - 5%, 0,5 - 5% et 1-5% en poids par rapport au poids total de la composition ophtalmique.

16. Composition selon l'une quelconque des revendications 13 à 15 comprenant un ou plusieurs produits ophtalmiques sélectionnés à partir :
- de produits anti-inflammatoires sélectionnés à partir de produits anti-inflammatoires stéroïdiens et non-stéroïdiens (AINS) ;
- de produis anti-allergiques, tout particulièrement sélectionnés à partir de FK506, 33-épi-chloro-33-désoxy-ascomycine, cromolyne, émadine, kétotifène, lévocabastine, lodoxamide, norkétotifène, clopatadine, et rizabène ;
- de produits destinés à traiter le glaucome (tout particulièrement, traitement de la pression intra-oculaire), tout particulièrement sélectionnés à partir de latanoprost, 15-kéto-latanoprost, unoprostone isopropyle, bétaxolol, clonidine, lévobunolol et timolole ;
- de produits anesthésiques, tout particulièrement sélectionnés à partir de chlorhydrate de cocaïne, de lidocaïne, d'oxybuprocaïne et de chlorhydrate de tétracaïne;
- de produits prévenant/inhibant la myopie;
- d'agents miotiques, tout particulièrement sélectionnés à partir de carbachol, pilocarpine et physostigmine ;
- d'inhibiteurs de l'anhydrase carbonique, tout particulièrement sélectionnés à partir d'acétazolamide et de dorzolamide ;
- d'agents alpha bloquant, tout particulièrement sélectionnés à partir d'apraclonidine et de brimonidine ; et
- d'anti-oxydants et/ou de vitamines, tout particulièrement sélectionnés à partir de l'acide ascorbique, du rétinol, de l'acétate de rétinol, du palmitate de rétinol et de tocophérols naturels et synthétique, tout particulièrement alfa-tocophérol et acétate d'alfa tocophérol ; et
- de matériaux biologiques, tout particulièrement peptides, protéines, ADN ou ARN.

17. Composition selon la revendication 16, **caractérisée en ce que** le produit ophtalmique est sélectionné à partir de produits anti-inflammatoires, de produits anti-allergiques et de produits destinés à traiter le glaucome.

18. Composition selon l'une quelconque des revendications 13 à 17, comprenant un ou plusieurs excipients acceptables d'un point de vue ophtalmique.

19. Composition selon la revendication 18, **caractérisée en ce que** les excipients comprennent un sel d'acide hyaluronique.

20. Composition selon la revendication 19, qui est substantiellement exempte de chlorure de benzalkonium.

21. Utilisation d'une composition comprenant un ou plusieurs produits pharmaceutiquement actifs, un composé de formule (I) et un véhicule comprenant un polymère en tant que système de dosage médicamenteux en vue d'une libération continue.

22. Utilisation selon la revendication 21, **caractérisée en ce que** le système de dosage médicamenteux destiné à une libération continue se présente dans un état semi-solide (aspect pâteux) ou, de préférence, ou dans un état solide.

23. Utilisation selon la revendication 21 ou 22, **caractérisée en ce que** le véhicule est une matrice composée d'un ou plusieurs polymères bioérodables et/ou d'un ou plusieurs polymères bioadhésifs.

24. Utilisation selon la revendication 23, **caractérisée en ce que** le polymère bioérodable est sélectionné à partir des acides polyhydroxy, tout particulièrement de l'acide polylactique et de l'acide polyglycolique ; des poly-esters, et des polyorthoesters, des polyanhydrides, des polycyanoacrylates, des gommes naturelles, tout particulièrement de la gomme d'acacia et de la gomme arabique; des celluloses ; et des (co)polymères (méth)acrylates.

25. Utilisation selon la revendication 23, ou de préférence selon la revendication 24, **caractérisée en ce que** la polymère bioadhésif est sélectionné à partir de la maltodextrine ; des celluloses ; des chitosans ; de l'acide hyaluronique ; des polyacrylates ; des polycarbophiles ; des polyvinylalcools et des polyvinylpyrrolidones.

26. Système de dosage médicamenteux destiné à une libération continue consistant en une composition qui comprend un ou plusieurs produits pharmaceutiquement actifs, un composé de formule (I) et un véhicule comprenant un polymère et sélectionnés à partir d'un film, d'un bâtonnet, d'une barre, d'une capsule, d'une protection pour la cornée, d'un anneau intra-cornéen, d'un implant, d'un insert, d'une lentille intra-oculaire, d'une lentille de contact thérapeutique, d'un comprimé, d'un mini comprimé, d'un mini disque et d'une pastille.

27. Méthode de conservation d'une composition pharmaceutique qui consiste à ajouter à ladite composition pharmaceutique une quantité efficace d'un conservateur sélectionné à partir des composés de formule (I) correspondants à la revendication 1 ; de préférence sélectionné à partir des composés de formule (Ia) correspondants à la revendication 2.

28. Utilisation d'un composé sélectionné à partir des composés de formule (I) correspondants à la revendication 1, tout particulièrement sélectionné à partir des composés de formule (Ia) selon la revendication 2, dans la préparation d'une composition pharmaceutique destinée à accroître la perméabilité d'un médicament contenu dans ladite composition pharmaceutique à travers les tissus de la peau, de la bouche, des muqueuses, des poumons, du vagin ou des yeux, et tout particulièrement, à travers les tissus conjonctifs, tout particulièrement destinée à accroître la perméabilité d'un produit contenu dans une composition ophtalmique à travers les tissus oculaires, tout particulièrement à travers les tissus de la cornée.

29. Utilisation selon la revendication 28, **caractérisée en ce que** ledit accroissement est un accroissement synergique.
